(19) Europäisches Patentamt
European Patent Office
Office européen des brevets

(11) Numéro de publication: **0 174 888**
**B1**

# (12) FASCICULE DE BREVET EUROPÉEN

(45) Date de publication du fascicule du brevet: **10.06.87**

(51) Int. Cl.⁴: **C 07 C 97/10**, A 61 K 31/135

(21) Numéro de dépôt: **85401610.2**

(22) Date de dépôt: **07.08.85**

(54) **Dérivés de 1-(aminophényl)-2-amino-éthanone, procédé de préparation et utilisation en thérapeutique.**

(30) Priorité: **20.08.84 FR 8412966**

(43) Date de publication de la demande: **19.03.86 Bulletin 86/12**

(45) Mention de la délivrance du brevet: **10.06.87 Bulletin 87/24**

(84) Etats contractants désignés: **AT BE CH DE FR GB IT LI LU NL SE**

(56) Documents cité:
**GB-A-453 437**

**CHEMICAL ABSTRACTS, vol. 47, no. 11, 10 juin 1953, col. 5380b-g, Columbus, Ohio, US; S.I. SERGIEVSKAYA et al.: "Synthesis of m-amino(hydroxy)phenyl -2-(methylamino)-ethanols and the catalytic reduction of m-nitroacetophenone"**
**CHEMICAL ABSTRACTS, vol. 51, no. 19, 10 octobre 1957, col. 14487h-i, Columbus, Ohio, US; I. SIMONYI et al.: "The reactions of aryl omega-aminoalkyl ketones with Fehling reagent"**

(73) Titulaire: **LABORATOIRE L. LAFON Société anonyme dite:, 1 rue Georges Médéric, F-94701 Maisons- Alfort (FR)**

(72) Inventeur: **Lafon, Louis, 5 rue de l'Alboni, F-75016 Paris (FR)**

(74) Mandataire: **Clisci, Serge, S.A. FEDIT- LORIOT CONSEILS EN PROPRIETE INDUSTRIELLE 38, avenue Hoche, F-75008 Paris (FR)**

EP 0 174 888 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1987

**Description**

La présente invention concerne en tant que produits industriels nouveaux des dérivés de 1-(amino-phényl)-2-amino-éthanone. Elle concerne également le procédé de préparation de ces nouveaux produits ainsi que leur utilisation en thérapeutique, en particulier en tant qu'agents antidépresseurs du système nerveux central (en abrégé SNC).

Les nouveaux dérivés selon l'invention sont caractérisés en ce qu'ils sont choisis parmi l'ensemble constitué par

(i) les 1-(aminophényl)-2-(alkylamino)-éthanones de formule

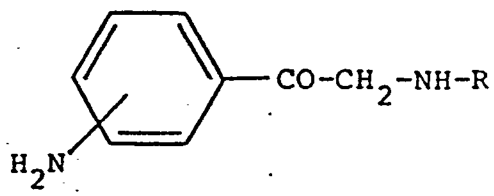

(I)

où R est $CH(CH_3)_2$ ou $C(CH_3)_3$; et

(ii) leurs sels d'addition.

Par sels d'addition, on entend ici les sels d'addition d'acide obtenus par réaction d'une base libre de formule I avec un acide minéral ou organique, d'une part, et les sels d'ammonium, d'autre part. Parmi les acides utilisables pour salifier les bases libres de formule I, on peut notamment mentionner les acides chlorhydrique, bromhydrique, acétique, formique, propionique, oxalique, fumarique, maléique, succinique, benzoïque, cinnamique, mandélique, citrique, malique, tartrique, aspartique, glutamique, méthanesulfonique, p-toluènesulfonique. Parmi les composés permettant d'obtenir des sels d'ammonium, on peut notamment citer $ICH_3$ et $ClCH_3$. D'une manière générale les sels d'addition d'acide sont préférés aux sels d'ammonium.

Compte tenu des définitions données ci-dessus le groupe $NH_2$ fixé sur le noyau benzénique peut être en position ortho, méta ou (de préférence) para. Le groupe R préféré est le groupe isopropyle.

De façon nullement limitative on a consigné dans le tableau I ci-après quelques composés typiques selon l'invention. Les composés les plus intéressants sont constitués par la 1-(4-aminophényl)-2-isopropyl-amino-éthanone et ses sels d'addition d'acide, en particulier le dichlorhydrate.

**Tableau I**

A-CO-$CH_2$-NH-R

| Produit | No. de code | A | R |
|---|---|---|---|
| Ex. 1(a) | - | 4-aminophényle | $CH(CH_3)_2$ |
| Ex. 2(b) | CRL 41 121 | 4-aminophényle | $CH(CH_3)_2$ |
| Ex. 3(c) | - | 3-aminophényle | $CH(CH_3)_2$ |
| Ex. 4(b) | - | 2-aminophényle | $C(CH_3)_3$ |
| Ex. 5(b) | - | 4-aminophényle | $C(CH_3)_3$ |
| Ex. 6(b) | - | 3-aminophényle | $C(CH_3)_3$ |

Notes

(a) base libre
(b) dichlorhydrate
(c) diméthanesulfonate

Pour préparer un composé de formule I on peut mettre en oeuvre une méthode connue en soi par application de mécanismes réactionnels classiques. Le procédé de préparation que l'on préconise selon l'invention consiste à soumettre un acétanilide de formule

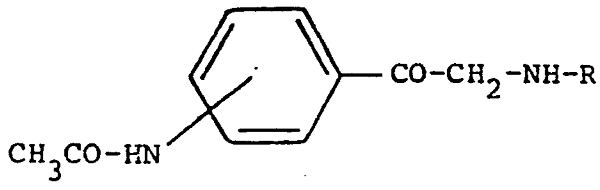

(II)

(où R est défini comme indiqué ci-dessus) à une réaction de désacétylation au moyen d'un acide concentré, à la température de reflux du milieu réactionnel pendant au moins 0,25 h.

Les composés selon l'invention présentent des propriétés thérapeutiques intéressantes vis-à-vis du SNC en raison des effets antidépresseurs et stimulants qu'ils manifestent. Leur profil neuropsychopharmacologique les rapproche des substances amphétaminiques. Ils se différencient cependant desdites substances amphétaminiques par une absence de toxicité particulière chez les souris groupées, voir à cet effet les résultats des essais relatifs au CRL 41 121 (produit de l'exemple 2) ci-après.

Selon l'invention on préconise une composition thérapeutique qui est caractérisée en ce qu'elle renferme, en association avec un excipient physiologiquement acceptable, un dérivé choisi parmi l'ensemble constitué par (i) les 1-(aminophényl)-2-(alkylamino)-éthanones de formule I et leurs sels d'addition non-toxiques, et (ii) leurs mélanges, en tant que principe actif.

Bien entendu, dans une telle composition, le principe actif intervient en quantité pharmaceutiquement efficace.

D'autres avantages et caractéristiques de l'invention seront mieux compris à la lecture qui va suivre, d'une part, d'exemples de préparation, et, d'autre part, de résultats d'essais pharmacologiques, l'ensemble de ces éléments n'étant nullement limitatif mais donné à titre d'illustration.

**Préparation I**

**Obtention du dichlorhydrate de 1-(4-aminophényl)-2-isopropylamino-éthanone.**

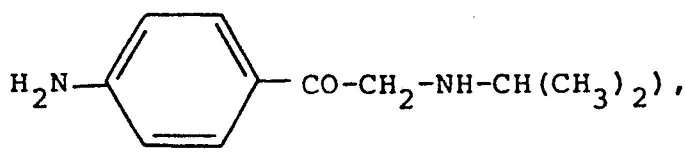

2HCl

(Exemple 2; No. de code: CRL 41 121).

On dissout 20 g de chlorhydrate de 1-(4-acétylamino-phényl)-2-isopropylamino-éthanone dans 200 ml d'acide HCl 4N, porte le milieu réactionnel à reflux pendant 0,25 h. Après évaporation sous vide aux 2/3 le dichlorhydrate attendu cristallise en paillettes de couleur jaune clair. Après filtration, lavage du précipité à l'eau glacée puis séchage, on obtient 17 g (rendement: 74 %) de CRL 41 121. $F_{inst.}$ = environ 260° C.

**Préparation II**

**Obtention de la 1-(4-aminophényl)-2-isopropylamino-éthanone,**

(Exemple 1)

On obtient la base libre ($F_{inst.}$ = 114° C) à partir du dichlorhydrate correspondant (CRL 41 121) par saponification.

On a résumé ci-après les résultats des essais qui ont été entrepris avec le CRL 41 121 (produit de l'exemple 2)

qui est le composé préféré selon l'invention sur le plan thérapeutique. Dans ces essais le CRL 41 121, en solution dans de l'eau distillée (à un pH de l'ordre de 2-3), a été sauf indication contraire administré par voie intrapéritonéale sous un volume de 20 ml/kg chez la souris mâle et de 5 ml/kg chez le rat mâle. Le pH varie en fonction de la concentration, en particulier il est de 2,5 pour une concentration de 1,6 g/l (dose de 32 mg/kg) et de 3 pour une concentration de 0,8 g/l (dose de 16 mg/kg).

**I. Toxicité**

Par voie intrapéritonéale chez la souris mâle, la DL-O (dose maximale non mortelle) est supérieure à 128 mg/kg, et la DL-100 est inférieure ou égale à 256 mg/kg.

**II. Comportement global et réactivités**

Des lots de trois animaux sont observés avant, puis 0,25 h, 0,50 h, 1 h, 2 h, 3 h, et 24 h après l'administration du CRL 41 121. On constate ce qui suit:

1°) chez la souris:

- aux doses de 1 mg/kg et 4 mg/kg:
- pas de symptôme particulier,
- à la dose de 16 mg/kg:
- une excitation (0,5 à 3 h),
- une augmentation de la réaction de peur (2h)
- une augmentation de la réactivité au toucher (3 h),
- a la dose de 64 mg/kg:
- une excitation (0,5 h à 3 h),
- une augmentation de la réaction de peur,
- une augmentation de la réactivité au toucher de 1 h (3 animaux sur 3) à 3 h (2 animaux sur 3),
- une légère hypothermie (de -2,2° C pour 2 animaux sur 3) 0,5 h après administration; et,

2°) chez le rat

- aux doses de 0,5 mg/kg et 2 mg/kg:
- pas de symptôme particulier,
- à la dose de 8 mg/g:
- une augmentation de l'agressivité,
- une augmentation de la réactivité au toucher,
- une mydriase 0,5 h après administration,
- à la dose de 32 mg/kg:
- une excitation (0,25 h à 2 h),
- des stéréotypies (0,5 h à 2 h),
- une stimulation de la respiration,
- une augmentation de la réaction de peur (0,25 h à 2 h),
- une augmentation de la réactivité au toucher (0,50 h à 2 h),
- une légère hyperthermie (+1,4° C) pendant 2 h,
- une mydriase durant pendant 2 h et maximale 0,5 h après administration.

**III. Action sur la température**

Des lots de 12 souris reçoivent le CRL 14 121 et on note la température rectale toutes les 30 minutes.

Aux doses de 16 mg/kg, 32 mg/kg et 64 mg/kg, on constate que le CRL 41 121 entraîne une faible hypothermie, maximale à 30 minutes et disparaissant dès 60 minutes. A dose plus élevée (128 mg/kg), aucune hypothermie n'est observée.

**IV. Recherche de mouvements stéréotypés**

Des lots de 6 rats reçoivent une injection intrapéritonéale de CRL 41 121 ou d'eau distillée immédiatement avant d'être placés dans des cages de dimensions réduites où leur comportement stéréotypé est noté toutes les 10 minutes jusqu'à extinction de l'effet.

On observe que le CRL 41 121 provoque, dès la dose de 8 mg/kg, l'apparition de mouvements stéréotypés chez le rat. L'intensité de cet effet croît avec la dose, et atteint à 16 mg/kg un niveau comparable à celui obtenu avec 2 mg/kg d'amphétamine.

## V. Interaction avec l'apomorphine

1°) Chez la souris

Des lots de 6 souris reçoivent le CRL 41 121 0,5 h avant l'injection sous-cutanée de 1 mg/kg ou 16 mg/kg d'apomorphine. On observe que le CRL 41 121 entraîne une hypothermie discrète à la plus forte dose utilisée (64 mg/kg) et antagonise (16 mg/kg et 64 mg/kg) l'hypothermie induite par l'apomorphine chez la souris, sans modifier l'attitude de verticalisation et les stéréotypies.

2°) Chez le rat

Le CRL 41 121 est administré à des lots de 6 rats 0,5 h avant l'injection sous-cutanée de 0,5 mg/kg d'apomorphine. On observe que le CRL 41 121, aux doses de 8 mg/kg et surtout 32 mg/kg, provoque une augmentation de l'index des stéréotypies induites par l'apomorphine chez le rat.

## VI. Interaction avec l'amphétamine

L'amphétamine (2 mg/kg) est injectée par voie intrapéritonéale à des lots de 6 rats 0,5 h après l'administration de CRL 41 121. On constate que le CRL 41 121, à la plus forte dose étudiée (32 mg/kg), provoque une potentialisation très importante des stéréotypies amphétaminiques.

## VII. Interaction avec l'halopéridol

Des lots de 6 rats reçoivent par voie intrapéritonéale 0,25 mg/kg d'halopéridol 0,5 h avant administration par voie intrapéritonéale de CRL 41 121 ou d'amphétamine.

On observe que l'halopéridol antagonise totalement les stéréotypies induites par l'amphétamine, d'une part, et le CRL 41 121 aux doses de 16 et 32 mg/kg, d'autre part.

## VIII. Interaction avec l'oxotrémorine

Le CRL 41 121 est administré à des lots de 6 souris 0,5 h avant l'injection intrapéritonéale de 0,5 mg/kg d'oxotrémorine.

1°) Action sur la température

Le CRL 41 121, dès la dose de 4 mg/kg, s'oppose à l'effet hypothermisant de l'oxotrémorine.

2°) Action sur les tremblements

Le CRL 41 121, à la plus forte dose étudiée (64 mg/kg) diminue faiblement l'intensité des tremblements dus à l'oxotrémorine.

3°) Action sur les symptômes cholinergiques périphériques

Le CRL 41 121 laisse inchangés les signes de stimulation cholinergique périphérique produits par l'oxotrémorine chez la souris (salivation, lacrymation, défécation).

## IX. Action sur le test des quatre plaques, la traction et l'électrochoc

Le test est pratiqué sur des lots de 10 souris 0,5 h après l'administration de CRL 41 121.

On observe que le CRL 41 121; aux doses de 16 mg/kg et 64 mg/kg entraîne une augmentation significative du nombre de passages punis, qu'il ne provoque pas d'incoordination motrice et qu'il s'oppose, à dose élevée (64 mg/kg), à l'effet convulsivant de l'électrochoc.

## X. Action sur la motilité spontanée

Une demi-heure après avoir reçu le CRL 41 121, les souris (6 par dose, 12 témoins) sont placées en actimètre où leur motilité est enregistrée pendant 30 minutes. On constate que le CRL 41 121, dès la dose de 4 mg/kg, entraîne une importante augmentation de l'activité motrice spontanée chez la souris.

### XI. Action sur l'agressivité intergroupes

Après avoir séjourné pendant 3 semaines dans chacune des moitiés d'une cage séparées par une cloison opaque, des groupes de 3 souris reçoivent le CRL 41 121. Une demi-heure plus tard, les deux groupes d'une même cage sont réunis par retrait de la cloison et on note le nombre de combats qui surviennent en 10 minutes.

On observe que, à la différence des substances amphétaminiques, le CRL 41 121 ne modifie pas l'agressivité intergroupes, que la dose utilisée soit excitante (64 mg/kg) ou non (1 mg/kg).

### XII. Action vis-à-vis de quelques comportements perturbés par divers agents

1°) Motilité réduite par habituation à l'enceinte

Après 18 heures de séjour dans les actimètres, les souris (6 par dose, 12 témoins) reçoivent le CRL 41 121. Elles sont aussitôt replacées dans leurs enceintes respectives et, une demi-heure plus tard, on enregistre leur motilité pendant 30 minutes.

On note que le CRL 41 121, dès la dose de 1 mg/kg provoque une reprise de l'activité chez la souris habituée à son enceinte.

2°) Motilité réduite par agression hypoxique

Une demi-heure après avoir reçu le CRL 41 121, les souris (10 par dose, 20 témoins) sont soumises à une anoxie hypobare aiguë [dépression de 600 mmHg (i.e. environ 8 x $10^4$ Pa) en 90 secondes, puis détente de 45 secondes], puis elles sont placées en actimètre où leur motilité est enregistrée pendant 10 minutes.

On constate que le CRL 41 121, aux plus fortes doses étudiées (16 mg/kg et 64 mg/kg), entraîne une amélioration de la récupération motrice chez les souris dont l'activité a été déprimée à la suite d'un bref séjour dans une enceinte à pression réduite.

3°) Anoxie asphyxique

Des lots de 10 souris reçoivent le CRL 41 121 une demi-heure avant l'administration intrapéritonéale de 32 mg/kg de triiodoéthylate de gallamine (substance curarisante de référence).

Le CRL 41 121 ne modifie pas le délai d'apparition des convulsions et de la mort consécutives à une anoxie asphyxique provoquée par un curarisant.

### XIII. Interaction avec le barbital

Une demi-heure après l'administration de CRL 41 121, des lots de 10 souris reçoivent une injection intrapéritonéale de barbital (220 mg/kg).

Dès la dose de 4 mg/kg, le CRL 41 121 diminue la durée du sommeil barbiturique. A la plus forte dose étudiée (64 mg/kg), le CRL 41 121 abolit l'effet du barbital.

### XIV. Action sur le "désespoir comportemental"

Une demi-heure après avoir reçu le CRL 41 121, des lots de 6 souris sont placés dans un bécher rempli d'eau sur une hauteur de 6 cm. On note la durée totale d'immobilité entre la 2ème et la 6ème minute suivant l'immersion.

On observe que le CRL 41 121 diminue nettement aux doses de 16 mg/kg et 64 mg/kg la durée de l'immobilité de "désespoir". Cet effet est encore présent à 4 mg/kg.

### XV. Recherche d'une toxicité particulière ches les souris groupées

Aussitôt après l'administration de CRL 41 121, les souris groupées par 10 sont placées dans des cages de petites dimensions. Le nombre d'animaux morts est noté toutes les heures pendant 4 heures, et au bout de 24 heures. La toxicité du CRL 41 121 est déterminée dans les mêmes conditions à raison d'une souris par cage.

On constate que le CRL 41 121 n'est pas plus toxique chez les souris isolées que chez les souris groupées.

**XVI. Interaction avec l'α-méthylturoisne**

Des lots de 6 rats reçoivent par voie intrapéritonéale 128 mg/kg d'α-méthyltyrosine 2,5 h avant l'injection intrapéritonéale de CRL 41 121 ou d'amphétamine.

On observe que l'α-méthyltyrosine (i) empêche presque totalement l'apparition des stéréotypies induites par l'amphétamine, mais (ii) entraîne une diminution en intensité et surtout en durée des stéréotypies induites par le CRL 41 121 (aux doses de 16 et 32 mg/kg) sans toutefois supprimer lesdites stéréotypies.

**XVII. Interaction avec la réserpine**

1°) Chez la souris

Quatre heures après l'injection intrapéritonéale de 2,5 mg/kg de réserpine, des lots de 6 souris reçoivent le CRL 41 121. On observe que, aux doses de 4 mg/kg, 16 mg/kg et 64 mg/kg, le CRL 41 121 s'oppose à l'hypothermie réserpinique; le ptôsis est antagonisé seulement à la plus forte dose étudiée (64 mg/kg).

2°) Chez le rat

Des lots de 6 rats reçoivent une injection intrapéritonéale de 4 mg/kg de réserpine, soit 4 h (T-4 h), soit 24 h (T-24 h) avant administration par voie intrapéritonéale de CRL 41 121 (aux doses de 16, et 32 mg/kg) ou de méthylphénidate (i.e. α-phényl-2-pipéridineacétate de méthyle).

On observe que les mouvements stéréotypés induits soit par le CRL 41 121 soit par le méthylphénidate sont pratiquement supprimés par l'administration préalable (T-4 h) de réserpine.

En revanche l'administration préalable (T-24 h) de réserpine diminue modérément en intensité, plus nettement en durée, les stéréotypies provoquées par le CRL 41 121 d'une part, et le méthylphénidate d'autre part.

**XVIII. Interaction avec l'association réserpine + α-Méthyltyrosine**

Des lots de 6 rats reçoivent par voie intrapéritonéale le CRL 41 121 ou le méthylphénidate 24 h (T-24 h) après l'administration intrapéritonéale de 4 mg/kg de réserpine et 2,5 h (T-2,5 h) après l'administration intrapéritonéale de 128 mg/kg d'α-méthyltyrosine.

On constate que l'administration combinée de réserpine (T-24 h) et d'α-méthyltyrosine (T-2,5 h) empêche l'apparition des mouvements stéréotypés induits par le CRL 41 121 (aux doses de 16 et 32 mg/kg), d'une part, et le méthylphénidate, d'autre part.

**XIX. Conclusions**

Il résulte des essais neuropsychopharmacologiques résumés ci-dessus que le CRL 41 121 présente par voie intrapéritonéale des effets antidépresseurs, d'une part, et des effets stimulants, d'autre part. De plus on note que le CRL 41 121 exerce des effets qui peuvent être liés à une stimulation α-adrénergique périphérique (objectivés par un antagonisme du ptôsis réserpinique, un antagonisme des tremblements induits par l'oxotrémorine, et une mydriase -cependant on observe que les animaux ne présentent ni piloérection ni salivation épaisse-).

Le blocage par l'halopéridol des stéréotypies induites par le CRL 41 12 implique que lesdites stéréotypies sont probablement consécutives à une stimulation d'un récepteur dopaminergique post-synaptique. Une action directe du CRL 41 121 sur ce récepteur semble devoir être exclue car des manipulations du système dopasinergique pré-synaptique [par exemple réserpine (T-4 h) ou réserpine (T-24 h) + α-méthyltyrosine (T-2,5 h) ches le rat] sont susceptibles d'empêcher l'apparition de ces stéréotypies. Comme par ailleurs 1'α-méthyltyrosine utilisée seule n'inhibe pas totalement les effets stéréotypigènes du CRL 41 121, alors qu'elle supprime les stéréotypies amphétaminiques (ce qui se traduit par une inhibition de la synthèse des catécholamines), on présume qu'au niveau du système dopaminergique le CRL 41-121

- libèrerait faiblement la dopasine nouvellement synthétisée (phénonène objectivé par l'inhibition incomplète par l'α-m-éthyltyrosine des stéréotypies induites ches le rat par le CRL 41-121), et
- inhiberait la recapture de dopamine avec pour conséquence une libération de dopamine d'un pool de réserve.

En bref, le mécanisme d'action du CRL 41 121 est différent de celui de l'apomorphine et de l'amphétamine, mais proche de celui du méthylphénidate ou de la nomifensine.

**XX. Etude neuropsychopharmacologique par voie gastrique**

Des essais complémentaires ont été entrepris, pour compléter les résultats sus-visés, en administrant le CRL 41 121 par voie gastrique, en solution dans de l'eau distillée, sous un volume de 20 ml/kg chez la souris mâle.

1°) Action sur la motilité spontanée

Selon le protocole décrit au point X ci-dessus, on constate que, dès la dose de 8 mg/kg, le CRL 41 121 augmemte fortement et de façon statistiquement significative l'activité motrice chez la souris. L'effet croît avec la dose et l'hypermotilité devient très importante à la dose de 128 mg/kg.

2°) Interaction avec le barbital

Selon le protocole décrit au point XIII ci-dessus, on constate que le CRL 41 121, dès la dose de 8 mg/kg, diminue la durée du sommeil barbiturique. L'effet devient maximal aux doses de 32 et 128 mg/kg.

3°) Interaction avec l'apomorphine

Des lots de 12 souris reçoivent le CRL 41 121 une demi-heure avant l'injection sous-cutanée de 16 mg/kg d'apomorphine. On observe que, aux plus fortes doses utilisées (32 et 128 mg/kg) le CRL 41 121 antagonise nettement l'action hypothermisante de l'apomorphine, sans modifier le comportement de verticalisation et les stéréotypies.

L'ensemble des essais neuropsychopharmacologiques réalisés par voie gastrique montre que les effets de type stimulant et/ou éveillant du CRL 41 121 apparaissent pour des doses supérieures et égales à 8 mg/kg. Par contre, ceux de type antidépresseur n'apparaissent qu'à partir de la dose de 32 mg/kg.

La dissociation en fonction des doses entre les effets de type stimulant et/ou éveillant, et ceux de type antidépresseur, observée après administration gastrique de CRL 41 121 est comparable à celle obtenue après administration intrapéritonéale.

En clinique on a obtenu de bons résultats en administrant le CRL 41 121 par voie orale (sous forme de comprimés ou de gélules) en tant qu'agent antidépresseur, dans le traitement des dépressions et des états dépressifs.

On recommande donc l'utilisation des substances de formule I et de leurs sels d'addition non-toxiques pour l'obtention d'un médicament antidépresseur du SNC destiné à une utilisation thérapeutique vis-à-vis des dépressions et des états dépressifs.

**Revendications**

pour les Etats contractants: BE, CH, DE, FR, GB, IT, LI, LU, NL, SE

1. Dérivé de 1-(aminophényl)-2-amino-éthanone, caractérisé en ce qu'il est choisi parmi l'ensemble constitué par

(i) les 1-(aminophényl)-2-(alkylamino)-éthanones de formule

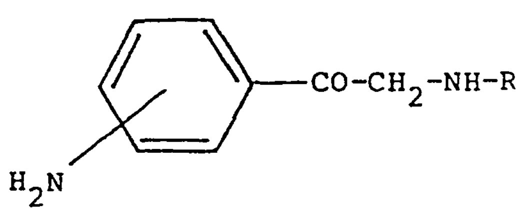

(I)

où R est $CH(CH_3)_2$ ou $C(CH_3)_3$; et,

(ii) leurs sels d'addition.

2. Dérivé selon la revendication 1, caractérisé en ce que le groupe $NH_2$ lié au noyau benzénique est en position para par rapport au groupe carbonyle.

3. Dérivé selon la revendication 1, caractérisé en ce que R est $CH(CH_3)_2$.

4. 1-(4-Aminophényl)-2-isopropylamino-éthanone ses sels d'addition.

5. Composition thérapeutique, caractérisée en ce qu'elle renferme, en association avec un excipient physiologiquement acceptable, un dérivé choisi parmi l'ensemble constitué par (i) les 1-(aminophényl)-2-(alkylamino)-éthanones de formule

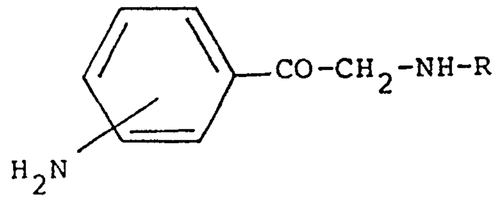

(I)

où R est $CH(CH_3)_2$ ou $CH(CH_3)_3$, et leurs sels d'addition non toxiques, et (ii) leurs mélanges.

6. Composition thérapeutique caractérisée en ce qu'elle renferme, en association avec un excipient physiologiquement acceptable, la 1-(4-aminophényl)-2-isopropylamino-éthanone ou l'un de ses sels d'addition non toxiques.

7. Procédé de préparation d'un dérivé de 1-(aminophényl)-2-(alkylamino)-éthanone de formule I selon la revendication 1, caractérisé en ce que l'on soumet à une réaction de désacétylation une 1-(acétylaminophényl)-2-(alkylamino)-éthanone de formule

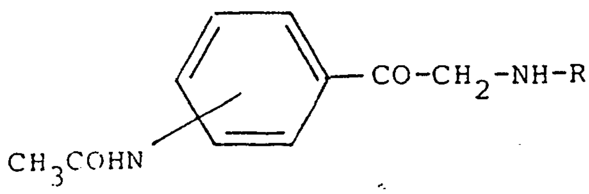

(II)

où R est $CH(CH_3)_2$ ou $C(CH_3)_3$, au moyen d'un acide, à la température de reflux du milieu réactionnel pendant au moins 0,25 h.

8. Utilisation d'une substance appartenant à la famille des dérivés de 1-(aminophényl)-2-(alkylamino)-éthanone pour l'obtention d'un médicament antidépresseur du système nerveux central destiné à une utilisation thérapeutique vis-à-vis des dépressions, ladite utilisation étant caractérisée en ce que ladite substance est choisie parmi les composés de formule

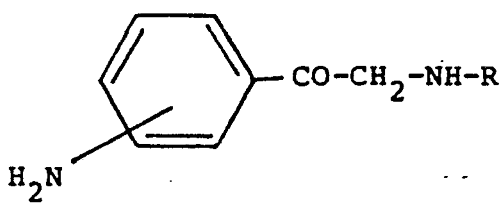

(I)

où R est $CH(CH_3)_2$ ou $C(CH_3)_3$, et leurs sels d'addition non-toxiques.

9. Utilisation d'une substance appartenant à la famille des dérivés de 1-(aminophényl)-2-(alkylamino)-éthanone pour l'obtention d'un médicament antidépresseur du système nerveux central destiné à une utilisation thérapeutique vis-à-vis des dépressions, ladite utilisation étant caractérisée en ce que ladite substance est choisie parmi la 1-(4-aminophényl)-2-isopropylamino-éthanone et ses sels d'addition non-toxiques.

**Revendications**

pour l'état contractant: AT

1. Procédé de préparation d'un dérivé de 1-(aminophényl)-2-amino-éthanone choisi parmi l'ensemble constitué par
(i) les 1-(aminophényl)-2-(alkylamino)-éthanones de formule

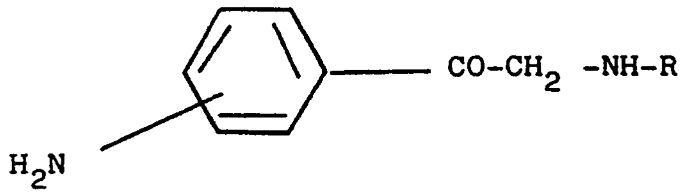

(I)

où R est $CH(CH_3)_2$ ou $C(CH_3)_3$; et (ii) leurs sels d'addition,
ledit procédé étant caractérisé en ce que l'on soumet à une réaction de désacétylation une 1-(acétyl-amino-phényl)-2-(alkylamino)-éthanone de formule

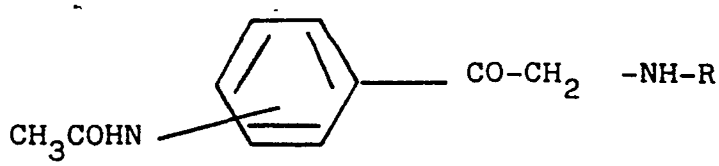

(II)

où R est $CH(CH_3)_2$ ou $C(CH_3)_3$ au moyen d'un acide, à la température de reflux du milieu réactionnel pendant au moins 0,25 h.

2. Procédé selon la revendication 1, caractérisé en ce que R est CH $(CH_3)_2$.

3. Procédé selon la revendication 1, caractérisé en ce que, pour préparer la 1-(4-aminophényl)-2-isopropylamino-éthanone et ses sels d'addition, on soumet la 1-(4-acétylaminophényl)-2-isopropylamino-éthanone à une réaction de désacétylation.

**Patentansprüche**

für den Vertragsstaaten BE, CH, DE, FR, GB, IT, LI, LU, NL, SE.

1. 1-(Aminophenyl)-2-amino-ethanon-Derivate, dadurch gekennzeichnet, daß sie aus der von (i) 1-(Aminophenyl)-2-(alkylamino)-ethanonen der Formel

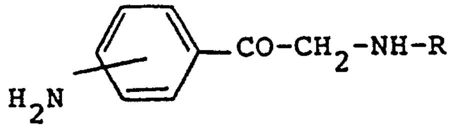

(I)

in der R = $CH(CH_3)_2$ oder $C(CH_3)_3$ ist, und (ii) ihren Additionssalzen gebildeten Gruppe ausgewählt sind.

2. Derivat nach Anspruch 1, dadurch gekennzeichnet, daß die am Benzolkern befindliche $NH_2$-Gruppe in p-Position, bezogen auf die Carbonylgruppe, ist.

3. Derivat nach Anspruch 1, dadurch gekennzeichnet, daß R = $CH(CH_3)_2$ ist.

4. 1-(4-Aminophenyl)-2-isopropylamino-ethanon und seine Additionssalze.

5. Therapeutische Zusammensetzung, dadurch gekennzeichnet, daß sie zusammen mit einem physiologisch verträglichen Exzipienten ein Derivat enthält, das aus der von (i) 1-(Aminophenyl)-2-(alkylamino)-ethanonen der Formel

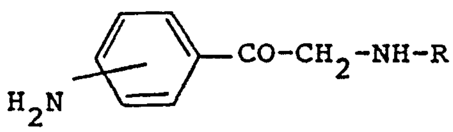

(I)

in der R = $CH(CH_3)_2$ oder $C(CH_3)_3$ ist, und ihren nicht-toxischen Additionssalzen und (ii) ihren Gemischen gebildet ist.

6. Therapeutische Zusammensetzung, dadurch gekennzeichnet, daß sie zusammen mit einem physiologisch verträglichen Exzipienten 1-(4-Aminophenyl)-2-isopropylamino-ethanon oder nicht-toxisches Additionssalz desselben enthält.

7. Verfahren zur Herstellung eines Derivates des 1-(Aminophenyl)-2-(alkylamino)-ethanons der Formel I nach Anspruch 1, dadurch gekennzeichnet, daß man ein 1-(Acetyl-aminophenyl)-2-(alkylamino)-ethanon der Formel

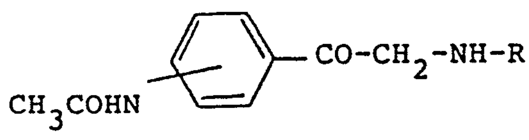

(II)

in der R $CH(CH_3)_2$ oder $C(CH_3)_3$ ist, mittels einer Säure bei der Rückflußtemperatur des Reaktionsgemisches mindestens 0,25 h deacetyliert.

8. Verwendung einer Substanz aus der Familie der Derivate des 1-(Aminophenyl)-2-(alkylamino)-ethanons zum Erhalt eines auf das zentrale Nervensystem antidepressiv wirkenden Medikaments, welches zu einer therapeutischen Verwendung bei Depressionen bestimmt ist, wobei die Verwendung dadurch gekennzeichnet ist, daß die genannte Substanz aus den Verbindungen der Formel

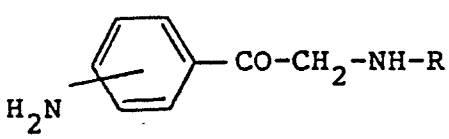

(I)

in der R $CH(CH_3)_2$ oder $C(CH_3)_3$ ist, und ihren nicht-toxischen Additionssalzen ausgewählt ist.

9. Verwendung einer Substanz aus der Familie der Derivate des 1-(Aminophenyl)-2-(alkylamino)-ethanons zum Erhalt eines Medikaments, das antidepressiv auf das zentrale Nervensystem wirkt und zur therapeutischen Anwendung bei Depressionen bestimmt ist, wobei die Verwendung dadurch gekennzeichnet ist, daß die

genannte Substanz aus 1-(4-Aminophenyl)-2-isopropylamino-ethanon und seinen nicht-toxischen Additionssalzen ausgewählt ist.

**Patentansprüche**

für den Vertragsstaat AT

1. Verfahren zur Herstellung eines Derivats des 1-(Aminophenyl)-2-amino-ethanons, ausgewählt aus der von (i) 1-(Aminophenyl)-2-(alkylamino)-ethanonen der Formel

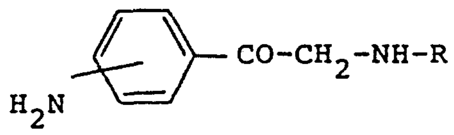

(I)

in der R $CH(CH_3)_2$ oder $C(CH_3)_3$ ist, und (ii) ihren Additionssalzen gebildeten Gruppe, dadurch gekennzeichnet, daß man ein 1-(Acetylamino-phenyl)-2-(alkylamino)-ethanon der Formel

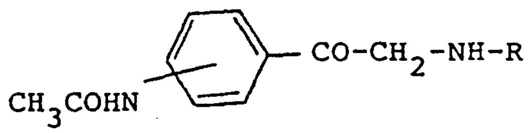

(II)

in der R $CH(CH_3)_2$ oder $C(CH_3)_3$ ist, mittels einer Säure bei der Rückflußtemperatur des Reaktionsgemisches mindestens 0,25 h einer Deacetylierungsreaktion unterwirft.

2. Verfahren nach Anspruch 1, dadurch gekennzeichnet, daß R = $CH(CH_3)_2$ ist.

3. Verfahren nach Anspruch 1, dadurch gekennzeichnet, daß man zur Herstellung von 1-(4-Aminophenyl)-2-isopropylamino-ethanon und seiner Additionssalze 1-(4-Acetylamino-phenyl)-2-isopropylamino-ethanon einer Deacetylierungsreaktion unterwirft.

**Claims**

for the contracting states: BE, CH, DE, FR, GB, IT, LI, LU, NL, SE.

1. A 1-(aminophenyl)-2-aminoethanone derivative selected from the group comprising:
(i) 1-(aminophenyl)-2-(alkylamino)ethanones of the formula:

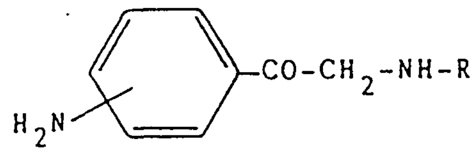

(I)

in which R is $CH(CH_3)_2$ or $C(CH_3)_3$; and
(ii) addition salts thereof.

2. A derivative according to claim 1, wherein the $NH_2$ group is in the para position on the phenyl nucleus, relative to the carbonyl group.

3. A derivative according to claim 1, wherein R is $CH(CH_3)_2$.

4. 1-(4-Aminophenyl)-2-isopropyleminoethanone and addition salts thereof.

5. A therapeutic composition comprising, in association with a physiologically acceptable excipient, a compound selected from the group comprising (i) 1-(aminophenyl)-2-(alkylamino)ethanones of the formula

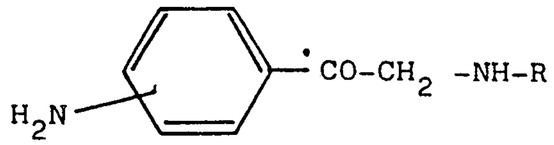

(I)

in which R is $CH(CH_3)_2$ or $C(CH_3)_3$
and non-toxic addition salts thereof, and (ii) mixtures thereof.

6. A therapeutic composition comprising, in association with a physiologically acceptable excipient, 1-(4-aminophenyl)-2-isopropylaminoethamone or one of its non-toxic addition salt.

7. A method for the preparation of a 1-(aminophenyl)-2-aminoethanone derivative of the formula I according to claim 1, which comprises subjecting a 1-(acetylaminophenyl-2-(alkylamino)ethanone of the formula:

(II)

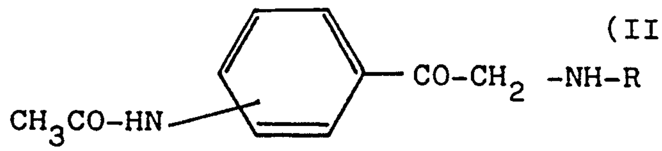

in which R is $CH(CH_3)_2$ or $C(CH_3)_3$, to a deacetylation reaction with an acid at the reflux temperature of the reaction medium for at least 0.25 hour.

8. A use of a substance belonging to the family of 1-(aminophenyl)-2-(alkylamino)-ethanone derivatives for obtaining an antidepressant medicament destined to a therapeutical use vis-à-vis depressions, characterized in that said substance is selected from the compounds of the formula

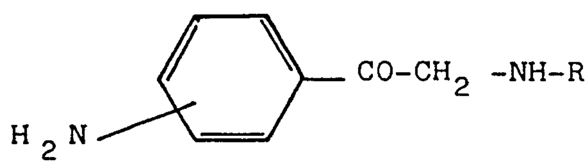

(I)

9. A use of a substance belonging to the family of 1-(aminophenyl)-2-(alkylamino)-ethanone derivatives for obtaining an antidepressant medicament destined to a therapeutical use vis-à-vis depressions, characterized in that said substance is selected from the 1-(4-aminophenyl)-2-isopropylamino-ethanone and its non-toxic addition salts.

**Claims**

for the contracting state: AT.

1. A method for the preparation of a 1-(aminophenyl)-2-aminoethanone derivative selected from the group comprising:

(i) 1-(aminophenyl)-2-(alkylamino)ethanones of the formula:

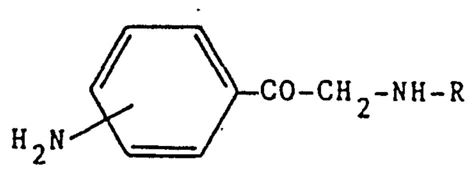 (I)

in which R is $CH(CH_3)_2$ or $C(CH_3)_3$; and
(ii) addition salts thereof, which comprises subjecting a 1-(acetylaminophenyl)-2-(alkylamino)ethanone of the formula:

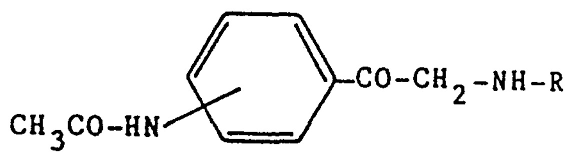 (II)

in which R is $CH(CH_3)_2$ or $C(CH_3)_3$ to a deacetylation reaction with an acid, at the reflux temperature of the reaction mixture, for at least 0.25 hour.

2. The method according to claim 1, wherein R is $CH(CH_3)_2$.

3. The method according to claim 1, for preparing the 1-(4-aminophenyl)-2-isopropylaminoethamone and its addition salts, which comprises subjecting the 1-(4-acetylaminophenyl)-2-isopropylamino-ethanone to a deacetylation reaction.